(19) Europäisches Patentamt
European Patent Office
Office européen des brevets

(11) Veröffentlichungsnummer: **0 309 844 B1**

(12) # EUROPÄISCHE PATENTSCHRIFT

(45) Veröffentlichungstag der Patentschrift: **04.12.91**

(51) Int. Cl.5: **C07C 329/02**

(21) Anmeldenummer: **88115298.7**

(22) Anmeldetag: **17.09.88**

(54) **Verfahren zur Herstellung von Chlorothiolformiaten.**

(30) Priorität: **22.09.87 DE 3731812**

(43) Veröffentlichungstag der Anmeldung:
**05.04.89 Patentblatt 89/14**

(45) Bekanntmachung des Hinweises auf die Patenterteilung:
**04.12.91 Patentblatt 91/49**

(84) Benannte Vertragsstaaten:
**AT BE CH DE ES FR GB IT LI NL**

(56) Entgegenhaltungen:
**US-A- 3 299 114**

(73) Patentinhaber: **BASF Aktiengesellschaft**
**Carl-Bosch-Strasse 38**
**W-6700 Ludwigshafen(DE)**

(72) Erfinder: **Decker, Martin, Dr.**
**Maria-Stuart-Strasse 21**
**W-6700 Ludwigshafen(DE)**
Erfinder: **Franzischka, Wolfgang, Dr.**
**Flomersheimer Strasse 36**
**W-6710 Frankenthal(DE)**
Erfinder: **Wache, Harro, Dr.**
**Raiffeisenstrasse 23**
**W-6701 Fussgoenheim(DE)**
Erfinder: **Franz, Dieter, Dr.**
**Horst-Schork-Strasse 75**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft ein verbessertes Verfahren zur Herstellung von Chlorothiolformiaten der allgemeinen Formel I

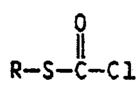

I

in der R einen organischen Rest bedeutet, durch Phosgenierung der entsprechenden Thiole II

R-SH II

in Gegenwart katalytischer Mengen einer cyclischen tertiären Stickstoffbase.

Die Herstellung der für zahlreiche organische Synthesen wichtigen Chlorothiolformiate I, die auch als Chlorothiolcarbonate oder S-Ester der Chlorthiokohlensäure bezeichnet werden, durch Phosgenierung der entsprechenden Thiole (Mercaptane) II

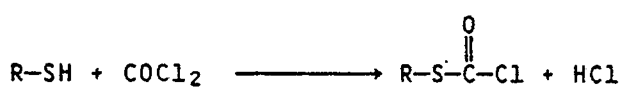

ist allgemein bekannt.

Da diese Reaktion relativ langsam und häufig auch nur unvollständig verläuft, wurde verschiedentlich empfohlen, sie in Gegenwart von Katalysatoren vorzunehmen.

Speziell ist es aus der US-A 3 299 114 bekannt, als Katalysatoren offenkettige oder heterocyclische tertiäre Amine oder heterocyclische aromatische Stickstoffbasen vom Typ des Pyridins zu verwenden. Die hierbei erzielten Ausbeuten an den Chlorothiolformiaten lassen jedoch zu wünschen übrig, und außerdem geben diese Basen insofern zu verfahrenstechnischen Schwierigkeiten Anlaß, als die aus ihnen bei der Phosgenierung gebildeten Hydrochloride im Reaktionsgemisch unlöslich sind und somit zusätzliche Trennoperationen erforderlich machen.

Der Erfindung lag daher die Aufgabe zugrunde, diesen Nachteilen abzuhelfen.

Demgemäß wurde ein Verfahren zur Herstellung von Chlorothiolformiaten der allgemeinen Formel I

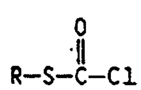

I

in der R einen organischen Rest bedeutet, durch Phosgenierung der entsprechenden Thiole II

R-SH II

in Gegenwart katalytischer Mengen einer cyclischen tertiären Stickstoffbase gefunden, welches dadurch gekennzeichnet ist, daß man als Basen III mono- oder dialkylsubstituierte 5- oder 6-gliedrige aromatische oder nicht-aromatische Heterocyclen mit 1 oder 2 tertiären N-Atomen verwendet, wobei die Summe der C-Atome in den Alkylresten 5 bis 20 beträgt.

Als Beispiele für derartige Basen III seien genannt:

- Alkylpyridine (IIIa), darunter besonders die 2-Alkylpyridine mit $C_6$-$C_{12}$-Alkylgruppen wie das 2-Undecylpyridin, sowie daneben die entsprechenden 3- und 4-Alkylpyridine und Dialkylpyridine wie das 2,6-Dibutylpyridin;
- N-Alkylpiperidine (IIIb) mit $C_6$-$C_{12}$-Alkylgruppen wie das N-Decylpiperidin und die C-Alkylderivate von N-Alkylpiperidinen, beispielsweise das N-Ethyl-2-dodecylpiperidin oder die entsprechenden 3- oder 4-alkylsubstituierten N-Alkylpiperidine;
- Alkylpyrimidine (IIIc) mit $C_6$-$C_{12}$-Alkylgrupopen wie das 2-n-Octylpyrimidin;
- 1-Alkyl-1,4,5,6-tetrahydropyrimidine (IIId) mit $C_6$-$C_{12}$-Alkylgruppen wie das 1-n-Hexyl-1,4,5,6-tetrahydropyrimidin sowie besonders die 1,2-Dialkyl-1,4,5,6-tetrahydropyrimidine mit insgesamt 5 bis 12 C-Atomen in den Alkylresten, darunter vor allem das 1-Methyl-2-isobutyl-1,4,5,6-tetrahydropyrimidin, das 1-Methyl-2-(1-ethylpentyl)-1,4,5,6-tetrahydropyrimidin und das 1,2-Diisobutyl-1,4,5,6-tetrahydropyrimidin. Diese und ähnliche 1,2-Dialkyl-1,4,5,6-tetrahydropyrimidine sind wegen ihrer leichten Zugänglichkeit durch Kondensation von 1,3-Diaminoalkanen und Alkylcarbonsäuren, z.B. nach dem in der EP-A 90 238 beschriebenen Verfahren, von besonderer wirtschaftlicher Bedeutung;

- Alkylpyrazine (IIIe) entsprechend den Alkylpyridinen IIIa;
- N,N′-Dialkylpiperazine (IIIf) mit insgesamt 5 bis 12 C-Atomen in den Alkylresten, beispielsweise N,N′-Diisobutylpiperazin;
- N-Alkylpyrrole (IIIg) mit $C_5$-$C_{12}$-Alkylgruppen, wie N-n-Octylpyrrol sowie 1,2- und 1,3-Dialkylpyrrole analog den Piperidinderivaten (IIIb);
- N-Alkylpyrrolidine (IIIh) entsprechend den Pyrrolderivaten IIIg;
- N-Alkylimidazole (IIIi) mit $C_5$-$C_{12}$-Alkylresten, wie N-Decylimidazol, sowie 1,2-, 1,4- und 1,5-Dialkylimidazole mit insgesamt 5 bis 12 C-Atomen in den Alkylrestne, beispielsweise 1-Methyl-2-isobutylimidazol, und
- N,N′-Dialkylimidazoline (IIIj) entsprechend den Piperazinderivaten IIIf.

Die definitionsgemäßen Basen III, darunter die Basen der Klassen IIIa bis IIIj, sind verhältnismäßig schwach. Sie bilden daher entweder keine Hydrochloride oder nur Amin-Hydrochloride mit sehr schwach ausgeprägtem salzartigen Charakter, was für die katalytische Wirkung dieser Basen vermutlich von Bedeutung ist. Ein weiteres wichtiges Kriterium für die Wirksamkeit dieser Basen im erfindungsgemäßen Verfahren ist, daß die Stickstoffatome einem Ringsystem angehören, und außerdem kommt es auf die definitionsgemäßen Alkylreste an, durch deren Anwesenheit die physikalische Affinität zwischen dem Katalysator und dem Thiol II erhöht wird.

Diese definitionsgemäßen Charakteristika der Basen III bedingen eine außergewöhnlich hohe katalystische Wirksamkeit, die bereits in Mengen von 0,01 mol% pro Mol II zu beobachten ist. Vorzugsweise setzt man die Basen III in Mengen von 0,05 bis 0,5 mol% von II ein, und bereits oberhalb von 1 mol% ergeben sich kaum noch wirtschaftliche Vorteile, so daß man nur in Ausnahmefällen, etwa zur Beschleunigung der Umsetzung von sehr reaktionsträgen Thiolen II größere Mengen - etwa bis zu 10 mol% von II - verwenden wird.

Da die Menge der Basen normalerweise nur sehr gering ist, ist es meistens nicht erforderlich, sie von den Verfahrensprodukten abzutrennen, denn bei den weiteren Reaktionen dieser Zwischenprodukte stören die Basen zumindest in geringen Konzentrationen im allgemeinen nicht. Hierin liegt ein weiterer Vorteil der erfindungsgemäß zu verwendenden Katalysatoren III.

Sieht man von den Basen III ab, gleicht die Phosgenierung derjenigen des Standes der Technik, wobei allerdings die hohe Wirksamkeit der Basen schonendere Bedingungen gestattet.

Für die Reaktionstemperaturen kommt im allgemeinen der Bereich von 0 bis 100 °C in Betracht, wobei sich Temperaturen zwischen 20 und 80 °C, vor allem 40 und 70 °C, besonders empfehlen.

Man führt die Umsetzung vorzugsweise unter Normaldruck aus, jedoch kann man auch bei höherem Druck - etwa bis zu 10 bar - arbeiten, z.B. wenn das Thiol II leicht flüchtig ist.

Die Mitverwendung von Lösungsmitteln ist im allgemeinen nicht erforderlich, es sei denn, das eingesetzte Thiol II und/oder das Verfahrensprodukt I wären unter den Reaktionsbedingungen nicht flüssig. Als Lösungsmittel oder auch als Medien für Reaktionen in Suspension eignen sich inerte organische Flüssigkeiten wie aliphatische und aromatische Kohlenwasserstoffe, beispielsweise Hexan und Toluol, chlorierte Kohlenwasserstoffe wie Methylenchlorid und Chlorbenzol, Ether wie Dioxan und Tetrahydrofuran und Lactame wie N-Methylpyrrolidon. Mit besonderem Vorteil verwendet man die Verfahrensprodukte I als Lösungsmittel, da sich in diesem Falle die Aufarbeitung der Reaktionsgemische am einfachsten gestaltet.

Im übrigen bietet die erfindungsgemäße Phosgenierung, die man wie üblich diskontinuierlich, halbkontinuierlich oder kontinuierlich ausführen kann, keine verfahrenstechnische oder apparativen Besonderheiten, so daß sich nähere Ausführungen hierzu erübrigen. Das gleiche gilt für die Aufarbeitung der Reaktionsgemische.

Nach den bisherigen Beobachtungen ist das erfindungsgemäße Verfahren von der Art der Thiole (II) nicht erkennbar abhängig, sieht man von den unterschiedlichen Reaktionsgeschwindigkeiten der Thiole, die auch bei den bisher bekannten Ausführungsformen dieser Reaktion festzustellen waren, ab.

Der Rest R in II bzw. I kann daher eine beliebige C-organische Gruppe sein, welche beliebige inerte Substituenten wie Fluor, Chlor, Brom, $C_1$-$C_4$-Alkoxygruppen, die Nitrogruppe, die Cyangruppe sowie Estergruppierungen enthalten kann.

Genannt seien:

- Alkyl-, Alkenyl- und Alkinylgruppen mit bis zu 20, vorzugsweise bis zu 12 C-Atomen;

- Cycloalkyl- und Cycloalkenylgruppen mit 4 bis 7 Ringgliedern
- Arylgruppen wie die Phenylgruppe, sowie
- gemischte Reste aus den vorgenannten Gruppen.

Selbstverständlich können auch mehrfunktionelle Thiole der erfindungsgemäßen Reaktion unterworfen werden.

Allgemein richtet sich die Art der Ausgangsstoffe (II) nach den gewünschten Chlorothiolformiaten (I), die ihrerseits als Zwischenprodukte für die Synthese von organischen Verbindungen, insbesondere von physiologischen Wirkstoffen wie Arznei- und Pflanzenschutzmitteln, dienen.

Beispiele 1 bis 5

Diskontinuierliche Herstellung von Chloro-n-octylthiolformiat (I/1)

In eine Mischung aus 292 g (2 mol) n-Octanthiol (n-Octylmercaptan) und 2 mmol einer Base III wurden bei T°C im Laufe von 90 bis 100 min 240 g (rd. 2,4 mol) Phosgen eingeleitet. Gleichzeitig wurde der entstandene Chlorwasserstoff von dem Reaktionsgemisch abgezogen. Anschließend wurde das Gemisch noch 1 h bei der Reaktionstemperatur gehalten.

Danach wurden überschüssiges Phosgen sowie restlicher Chlorwasserstoff mit Stickstoff aus dem Gemisch geblasen. Zurück blieb jeweils eine Masse, die zu p Gew.% aus (I/1) bestand, welches sich in einer Ausbeute von y % gebildet hatte.

Einzelheiten zu diesen Versuchen sind der nachstehendenb Tabelle zu entnehmen.

| Beispiel | Base III | T [°C] | p [Gew.%] | y [%] |
|---|---|---|---|---|
| 1 | 1-Isobutyl-2-methyl-1,4,5,6-tetrahydropyrimidin | 70-75 | 98,6 | 97,0 |
| 2 | 1-Methyl-2-(1-ethylpentyl)-1,4,5,6-tetrahydropyrimidin | 65-70 | 98,6 | 96,5 |
| 3 | 1,2-Diisobutyl-1,4,5,6-tetrahydropyrimidin | 75-80 | 99,3 | 98,8 |
| 4 | N-Decylimidazol | 60 | 99,3 | 99,0 |
| 5 | 2-Undecylpyridin | 60 | 99,3 | 99,1 |

Beispiele 6 und 7

Kontinuierliche Herstellung von Chloro-n-octylthiolformiat (I/1)

60 g/h (rd. 0,6 mol) Phosgen und 73 g/h (rd. 0,5 mol) n-Octanthiol, welches 0,1 mol% einer Base III enthielt, wurden in ein erstes Reaktionsgefäß geleitet, welches 360 g I/1 sowie 0,1 mol% der Base III enthielt. Vom ersten Gefäß wurde das Reaktionsgemisch nach Maßgabe des Zulaufs in ein zweites Gefäß geleitet, das mit 240 g der I/1-Lösung beschickt war.

Im ersten Gefäß wurde die Temperatur auf $T_1$ und im zweiten auf $T_2$ °C gehalten. Die Ausbeute an I/1 betrug y %.

Einzelheiten sind der nachstehenden Tabelle zu entnehmen.

| Beispiel | Base III | $T_1$ [°C] | $T_2$ [°C] | y [%] |
|---|---|---|---|---|
| 6 | 1,2-Diisobutyl-1,4,5,6-tetrahydropyrimidin | 63 | 66 | >99 |
| 7 | N-Decylimidazol | 58-63 | 63-65 | >99 |

Beispiele 8 bis 13

Diskontinuierliche Herstellung verschiedener Chlorothiolformiate

Auf die für die Beispiele 1 - 5 angegebene Weise wurde jeweils 1 mol eines Thiols II in Gegenwart von 1 mmol einer Base III im Laufe von t min bei T °C mit 1,2 mol Phosgen umgesetzt und noch 1 bis 2

Stunden zur Nachreaktion bei T °C gehalten. Die hierbei erhaltenen Massen wurden mit Stickstoff vom überschüssigen Phosgen sowie vom restlichen Chlorwasserstoff befreit. Sie bestanden im wesentlichen aus dem entsprechenden Chlorthiolformiat I, welches sich in einer Ausbeute von y % gebildet hatte.

Einzelheiten zu diesen Versuchen sind der nachstehenden Tabelle zu entnehmen.

| Beispiel | II, R-SH R | III | t [min] | T [°C] | III, R-S-CO-Cl y [%] |
|---|---|---|---|---|---|
| 8 | n-Hexyl | 2-Undecylpyridin | 35 | 60 | 98 |
| 9 | n-Hexyl | N-Decylimidazol | 35 | 50 | 99 |
| 10 | Cyclohexyl | N-Decylimidazol | 60 | 50 | 98 |
| 11 | Cyclohexyl | 2-Undecylpyridin | 75 | 50 | 98 |
| 12 | n-Dodecyl | 1,2-Diisobutyltetrahydropyrimidin | 60 | 60 | 99 |
| 13 | n-Dodecyl | 2-Undecylpyridin | 35 | 67 | 99 |

## Patentansprüche

1. Verfahren zur Herstellung von Chlorothiolformiaten der allgemeinen Formel I

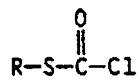

I

in der R einen organischen Rest bedeutet, durch Phosgenierung der entsprechenden Thiole II

R-SH II

in Gegenwart katalytischer Mengen einer cyclischen tertiären Stickstoffbase, dadurch gekennzeichnet, daß man als Basen III mono- oder dialkylsubstituierte 5- oder 6-gliedrige aromatische oder nicht-aromatische Heterocyclen mit 1 oder 2 tertiären N-Atomen verwendet, wobei die Summe der C-Atome in den Alkylresten 5 bis 20 beträgt.

## Claims

1. A process for preparing a chlorothiolformate of the formula I

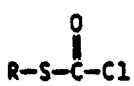

I

where R is an organic radical, by phosgene treatment of the corresponding thiol II

R-SH II

in the presence of catalytic amounts of a cyclic tertiary nitrogen base, which comprises using as bases III mono-or dialkyl-substituted 5- or 6-membered aromatic or nonaromatic heterocycles with 1 or 2 tertiary nitrogen atoms, where the total of alkyl carbon atoms is from 5 to 20.

## Revendications

1. Procédé de préparation de chlorothiolformiates de formule générale I

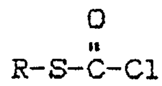

I

dans laquelle R représente un reste organique, par phosgénation des thiols II correspondants

R-SH II

en présence de quantités catalytiques d'une base azotée tertiaire cyclique, caractérisé en ce qu'on utilise, comme bases III, des hétérocycles aromatiques ou non aromatiques penta- ou hexagonaux à 1 ou 2 atomes d'azote tertiaire mono- ou dialkylsubstitués, la somme des atomes de carbone dans les restes alkyle étant comprise entre 5 et 20.